(19) Europäisches Patentamt
European Patent Office
Office européen des brevets

(11) Numéro de publication: **0 570 577 B1**

(12) # FASCICULE DE BREVET EUROPEEN

(45) Date de publication du fascicule du brevet: **15.11.95**

(51) Int. Cl.$^6$: **A61K 7/00**

(21) Numéro de dépôt: **93902284.4**

(22) Date de dépôt: **03.12.92**

(86) Numéro de dépôt internationale :
**PCT/FR92/01134**

(87) Numéro de publication internationale :
**WO 93/10745 (10.06.93 93/14)**

Le dossier contient des informations techniques présentées postérieurement au dépôt de la demande et ne figurant pas dans le présent fascicule.

(54) **HUILE COSMETIOUE FILTRANTE CONTENANT UN FILTRE ORGANOPOLYSILOXANE ET D'AUTRES SILICONE A GROUPE SPECIFIOUE.**

(30) Priorité: **05.12.91 FR 9115101**

(43) Date de publication de la demande:
**24.11.93 Bulletin 93/47**

(45) Mention de la délivrance du brevet:
**15.11.95 Bulletin 95/46**

(84) Etats contractants désignés:
**DE ES FR GB IT**

(56) Documents cités:
**EP-A- 0 197 485** **EP-A- 0 330 369**
**EP-A- 0 335 777** **EP-A- 0 389 337**
**EP-A- 0 392 882** **EP-A- 0 392 883**
**EP-A- 0 424 260** **FR-A- 2 657 351**

(73) Titulaire: **L'OREAL**
**14, rue Royale**
**F-75008 Paris (FR)**

(72) Inventeur: **FORESTIER, Serge**
**16, allée Ferdinand-Buisson**
**F-77410 Claye-Souilly (FR)**
Inventeur: **HANSENNE, Isabelle**
**156-158, rue Legendre**
**F-75017 Paris (FR)**

(74) Mandataire: **Casalonga, Axel et al**
**BUREAU D.A. CASALONGA - JOSSE**
**Morassistrasse 8**
**D-80469 München (DE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 570 577 B1

Rank Xerox (UK) Business Services
(3.10/3.09/3.3.3)

Drug and Cosmetic Industry, vol. 138, no. 2, février 1986, R.L. GOLDEMBERG et al.: "Silicones in clear formulations", pages 34,38,40,44, voir page 40, colonne 2,: "Clear suntan oil"

Seifen-Öle-Fette-Wachse, vol. 114, no. 7, 21 avril 1988, (Augsburg, DE), J. ROIDL: "Silicone für moderne Hautpflegepräparate", pages 238-240, voir page 240: "Skin cleanser with sunscreen"

## Description

La présente invention a pour objet une huile cosmétique filtrante, transparente et photostable aux propriétés tactiles améliorées, destinée à protéger les matières kératiniques, et notamment la peau et les cheveux, contre les effets néfastes du rayonnement ultraviolet, contenant au moins un organopolysiloxane filtrant les UV particulier associé à un mélange constitué d'une silicone volatile et d'une huile de silicone de viscosité cinématique supérieure à $10^{-1}$ $m^2/s$ ou d'une gomme de silicone.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage; ce rayonnement UV-B doit donc être filtré.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques.

Il est souhaitable que les compositions filtrant les UV présentent, outre un bon indice de protection dans l'UV-A et l'UV-B, de bonnes propriétés cosmétiques telles que la douceur, sans effet gras ni collant et qu'elles s'étalent facilement, tout en possédant une bonne persistance, c'est-à-dire une bonne stabilité de l'indice de protection au cours du temps.

L'indice de protection ou IP peut s exprimer par le rapport du temps d'irradiation nécessaire pour atteindre le seuil érythématogène avec le filtre UV au temps nécessaire pour atteindre le seuil érythématogène sans filtre UV.

Il est également agréable de pouvoir disposer de compositions transparentes quand il s'agit d'huiles solaires.

On a déjà proposé d'utiliser des filtres liposolubles dérivés de benzophénone, de benzotriazole ou de benzylidènecamphre dans des compositions filtrantes à base d'huiles et notamment de silicones conventionnelles, c'est-à-dire des polydiméthylsiloxanes de faible viscosité, mais les huiles solaires qui en résultent sont pauvres en filtres UV en raison des faibles compatibilité ou solubilité des filtres dans les huiles.

On connaît, par ailleurs, des polymères filtrant le rayonnement ultraviolet, à chaîne hydrocarbonée ou siloxanique, porteurs de groupements chromophores divers.

Quelques polymères filtrants à chaîne siloxanique que l'on conviendra d'appeler "silicones filtres" dans la suite du texte, sont décrits comme étant plus solubles que les filtres conventionnels dans les huiles de silicone de faible viscosité.

Cependant, les filtres conventionnels ou lesdites silicones filtres, lorsqu'ils sont utilisés dans les huiles de silicone conventionnelles (polydiméthylsiloxanes de faible viscosité) manquent de stabilité et les qualités cosmétiques des compositions filtrantes obtenues sont insatisfaisantes : le toucher qu'elles conferent à la peau est gras et collant, partois glissant, et leur étalement est difficile. Les polymères filtrants à chaîne hydrocarbonée, quant à eux, sédimentent dans les milieux huileux siliconés.

La demanderesse a découvert que l'on pouvait obtenir une huile très filtrante, tout à fait transparente et stable dans le temps, et offrant des qualités cosmétiques exceptionnelles caractérisées par une douceur satinée sans effet collant ni gras, et une grande facilité à l'étalement en associant au moins un organopolysiloxane filtrant les UV, choisi parmi ceux à groupements dérivés du benzylidènecamphre, de benzalmalonate, de benzophénone et de benzotriazole à un mélange de deux composants (A) et (B) constitué de :

(A) une silicone volatile

(B) une huile de silicone de viscosité cinématique supérieure à $10^{-1}$ $m^2/s$ ou une gomme de silicone.

Une huile filtrante aux critères selon l'invention est de préférence une huile dans laquelle le mélange (A + B) est défini par un rapport pondéral B/A compris entre 0,07 et 0,2.

Les huiles filtrantes selon l'invention présentent par ailleurs une bonne photostabilité et une persistance améliorée par rapport aux huiles de l'art antérieur renfermant des filtres conventionnels.

Les silicones filtres utilisées selon l'invention sont par exemple des diorganopolysiloxanes comportant dans leur molécule au moins une unité de formule :

$$X - \overset{\overset{R_a}{|}}{Si} - O_{\frac{3-a}{2}} \qquad (I)$$

dans laquelle :

R désigne un groupe hydrocarboné saturé ou insaturé en $C_1$-$C_{30}$, un groupe hydrocarboné halogéné en $C_1$-$C_8$ ou un groupe triméthylsilyloxy;

a = 1 ou 2 ;

X = - A - Y

où A représente un radical divalent hydrocarboné aliphatique ou aromatique comportant au moins 2 atomes de carbone et renfermant éventuellement un ou plusieurs atomes d'oxygène;

Y représente le reste d'une molécule filtrant le rayonnement ultraviolet du type benzylidènecamphre, benzalmalonate, benzotriazole ou benzophénone.

En plus des unités de formule (I), le diorganopolysiloxane peut comporter des unités de formules (II) et/ou (III) :

$$R_b - Si - O_{\frac{4-b}{2}} \quad (II) \quad ; \quad Z - \overset{\overset{R_a}{|}}{Si} - O_{\frac{3-a}{2}} \quad (III)$$

dans lesquelles R a la même signification que dans la formule (I),

b est un nombre entier désignant 1, 2 ou 3;

a est un nombre entier désignant 1 ou 2;

Z = OY, Y ayant la même signification que dans la formule (I).

A titre de groupe hydrocarboné, on peut citer les radicaux alkyle en $C_1$-$C_{30}$, alcényle en $C_2$-$C_{30}$, cycloalkyle ou aromatique comme phényle ou tolyle.

A titre de groupe hydrocarboné halogéné, on peut citer le radical 3,3,3-trifluoropropyle.

Dans le diorganopolysiloxane constitué de motifs (I) et éventuellement (II) ou (III), au moins 40% en nombre des radicaux R sont des radicaux méthyle. Le nombre total des unités (I), (II) et (III) est de préférence inférieur ou égal à 250 et est compris en particulier entre 2 et 50.

Y représente un reste :

- benzylidènecamphre éventuellement substitué sur le noyau benzénique par des radicaux hydroxy, alkyle en $C_1$-$C_6$ ou alcoxy en $C_1$-$C_6$;
- benzalmalonate de dialkyle en $C_1$-$C_8$, éventuellement substitué sur le noyau benzénique par des radicaux hydroxy, alkyle en $C_1$-$C_6$ ou alcoxy en $C_1$-$C_6$;
- 2-(2'-hydroxyphényl)benzotriazole portant éventuellement sur l'un des noyaux aromatiques des substituants alkyle en $C_1$-$C_8$,halogène, alcoxy, carboxy, hydroxy, amino ou tétraalkylpipéridyle;
- 2-hydroxybenzophénone portant éventuellement d'autres substituants : alkyle en $C_1$-$C_8$, alcoxy en $C_1$-$C_8$, alcényloxy en $C_2$-$C_8$ ou hydroxy.

De tels polymères filtres à chaîne siloxanique sont décrits par exemple dans les demandes de brevet européen n° 0335777, 0392882, 0388218, 0392883 et 0389337, dans les brevets américains n° 4.696969 et n° 4.859759 et dans la demande de brevet français n° 2 657 351.

La silicone filtre est généralement présente dans les huiles filtrantes selon l'invention à une concentration totale comprise entre 0,5 et 13%, et de préférence entre 1 et 8% en poids, par rapport au poids total de la composition.

Les silicones volatiles (A) du mélange (A + B) selon l'invention peuvent être choisies parmi :

(i) les silicones cycliques comportant de 3 à 7 atomes de silicium et de préférence 4 à 5. Il s'agit, par exemple, de l'octaméthylcyclotétrasiloxane vendu sous le nom de VOLATILE SILICONE 7207 par UNION CARBIDE ou SILBIONE 70045 V 2 par RHONE POULENC, le décaméthylcyclopentasiloxane vendu sous le nom de VOLATILE SILICONE 7158 par UNION CARBIDE, SILBIONE 70045 V 5 par RHONE POULENC, ainsi que leurs mélanges;

(ii) les silicones volatiles linéaires ayant 2 à 9 atomes de silicium et possédant une viscosité inférieure ou égale à $5.10^{-6}$ $m^2/s$ à 25 °C. Il s'agit, par exemple, de l'hexaméthyldisiloxane vendu sous la dénomination SILBIONE 70 041 V 0,65 par la Société RHONE POULENC, du décaméthyltétrasiloxane vendu sous la dénomination SH 200 par la Société TORAY SILICONE ou des polyméthylphénylsiloxanes volatils tels que le produit SILICONOL AS vendu par la Société WACKER. Des silicones entrant dans cette classe sont également décrites dans l'article publié dans Cosmetics and Toiletries, Vol. 91, Jan. 76, p. 27-32 - TODD & BYERS "Volatile Silicone fluids for cosmetics".

Les huiles de silicone de viscosité supérieure à $10^{-1}$ m²/s peuvent être choisies parmi les polydiorganosiloxanes à groupements terminaux triméthylsilyle de viscosité $2x10^{-1}$ m²/s à $25x10^{-1}$ m²/s à 25°C.

Parmi ces huiles, on peut citer l'huile 47 V 500.000 vendue par RHONE POULENC.

Les gommes de silicone, utilisées selon la présente invention, sont des polydiorganosiloxanes de fortes masses moléculaires, comprises entre 200.000 et 1.000.000, utilisées seules ou en mélange dans un solvant choisi parmi les silicones volatiles telles que définies ci-dessus.

On cite, par exemple, les gommes suivantes :

- poly[(diméthylsiloxane)/(méthylvinylsiloxane)],
- poly[(diméthylsiloxane)/(diphénylsiloxane)], éventuellement à groupements terminaux diméthylvinylsilyloxy,
- poly[(diméthylsiloxane)/(phénylméthylsiloxane)],
- poly[(diméthylsiloxane)/(diphénylsiloxane)/(méthylvinylsiloxane)].

On peut citer, par exemple, à titre non limitatif, les mélanges suivants :

. les mélanges formés à partir d'un polydiméthylsiloxane hydroxylé en bout de chaîne (DIMETHICONOL selon la nomenclature CTFA) et d'un polydiméthylsiloxane cyclique (CYCLOMETHICONE selon la nomenclature CTFA), tel que le produit Q2-1401 vendu par la Société DOW CORNING,

. les mélanges formés à partir d'une gomme polydiméthylsiloxane avec une silicone cyclique, tel que le produit SF 1214 Silicone Fluid de la Société GENERAL ELECTRIC (qui est une gomme SE 30, correspondant à une diméthicone, ayant un poids moléculaire de 500.000 solubilisée dans la silicone SF 1202 Silicone Fluid (correspondant au décaméthylcyclopentasiloxane)).

La concentration du mélange (A + B) de silicone volatile (A) et d'huile de silicone de viscosité supérieure à $10^{-1}$ m²/s ou de gomme de silicone (B) est avantageusement comprise entre 67 et 99,5%, et de préférence entre 75 et 80% en poids, par rapport au poids total de la composition, le rapport pondéral B/A étant compris entre 0,07 et 0,2.

La viscosité cinématique des huiles filtrantes selon l'invention mesurée au Contrav VE module 2 est comprise entre $10^{-4}$ et $7x10^{-4}$ m²/s et de préférence entre $2x10^{-4}$ et $5,5x10^{-4}$ m²/s.

Une huile cosmétique filtrante préférée selon l'invention renferme, outre la silicone filtre et un mélange (A + B) tel que défini ci-dessus, un mélange de solvants organiques dans une proportion pondérale inférieure à 20% par rapport à la composition totale, constitué par un mélange d'alcool éthylique et d'un ester pouvant être choisi parmi un benzoate d'alcools en $C_{12}$-$C_{15}$, un palmitate d'isopropyle, un myristate d'isopropyle, un monococoate de 2-éthylhexyle, du dipélargonate de propylèneglycol, un ester d'acides gras ramifiés et d'alcools gras saturés en $C_{16}$-$C_{18}$ (CETIOL SN DEO d'HENKEL), un ester d'acides gras et de glycérol (SOFTISAN 649 de HULS), un ester d'acides gras (caprylique-caprique) et de propylèneglycol, ou les mélanges de ces composés.

L'alcool éthylique est de préférence compris dans ce mélange de solvants pour un tiers pondéral.

Un mélange plus particulièrement préféré est un mélange d'alcool éthylique/benzoate d'alcools en $C_{12}$-$C_{15}$.

L'huile cosmétique filtrante selon l'invention peut contenir en outre divers adjuvants habituellement utilisés en cosmétique pour ce type de composition sous réserve qu'ils n'en altèrent pas la transparence et que la viscosité finale demeure comprise dans les limites indiquées ci-dessus.

Ces adjuvants peuvent être à titre d'exemple, des adoucissants, des humectants, des vitamines, des huiles, des cires, des colorants, des conservateurs, des parfums et éventuellement des filtres UV-A et/ou UV-B.

Ils peuvent être introduits dans des proportions pondérales comprenant les solvants ne dépassant pas 20% du poids total des compositions.

L'huile cosmétique filtrante selon l'invention peut, bien entendu, servir de base à la préparation d'émulsions solaires H/E ou E/H.

L'invention vise aussi une émulsion cosmétique filtrante du type huile-dans-eau ou eau-dans-huile comprenant, à titre de phase grasse, l'huile cosmétique filtrante telle que définie ci-dessus.

L'invention a également pour objet un procédé de protection de l'épiderme humain et des cheveux contre le rayonnement ultraviolet consistant à appliquer sur la peau ou les cheveux une quantité efficace de l'huile cosmétique filtrante ci-dessus.

L'invention sera mieux illustrée par les exemples non limitatifs ci-après.

EXEMPLE 1

On prépare une huile solaire transparente de composition suivante :

- Polydiméthylsiloxane à greffon 4'-triméthylèneoxy 3-benzylidènecamphre de formule : 6 g

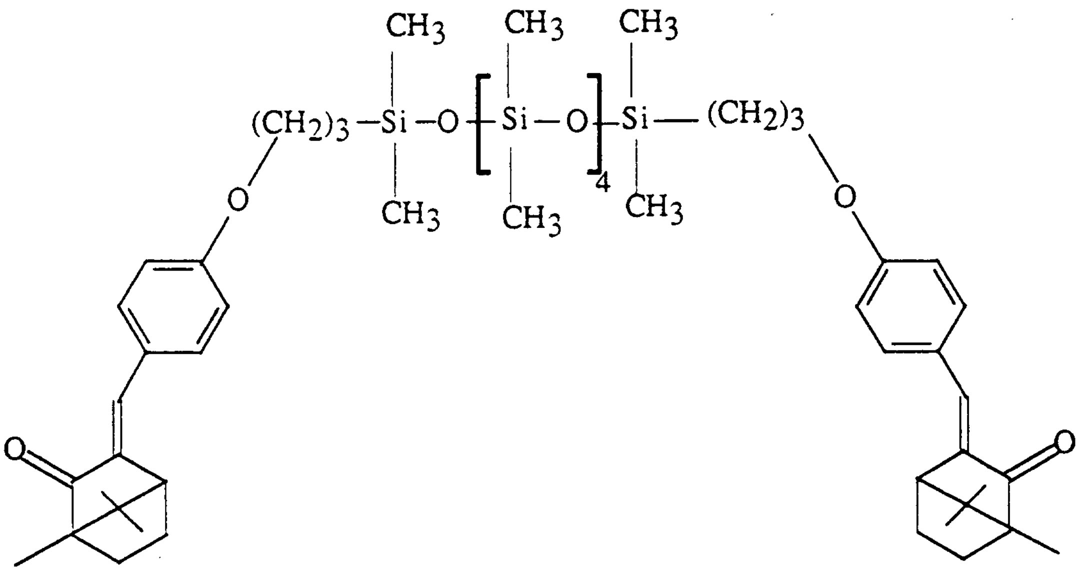

selon la demande EP 0335777 de la demanderesse.

| | | | |
|---|---|---|---|
| - Décaméthylcyclopentasiloxane | | 20 | g |
| - Mélange de diméthiconol (13%), d'octaméthylcyclotétrasiloxane et de décaméthylcyclopentasiloxane (87%), vendu sous la dénomination Q2-1401 par la Société DOW CORNING | | 59,4 | g |
| - Benzoate d'alcools en $C_{12}$-$C_{15}$ vendu sous la dénomination FINSOLV TN par la Société WITCO | | 7,9 | g |
| - Huile de tournesol | | 0,5 | g |
| - Beurre de karité | | 0,1 | g |
| - Acétate de vitamine E | | 0,5 | g |
| - Conservateurs | qs | | |
| - Parfum | qs | | |
| - Alcool éthylique absolu | qsp | 100 | g |

EXEMPLE 2

On prépare une huile solaire transparente de composition suivante :

| | | | |
|---|---|---|---|
| - Polydiméthylsiloxane à greffon dérivé de benzotriazole de formule : | | 6 | g |

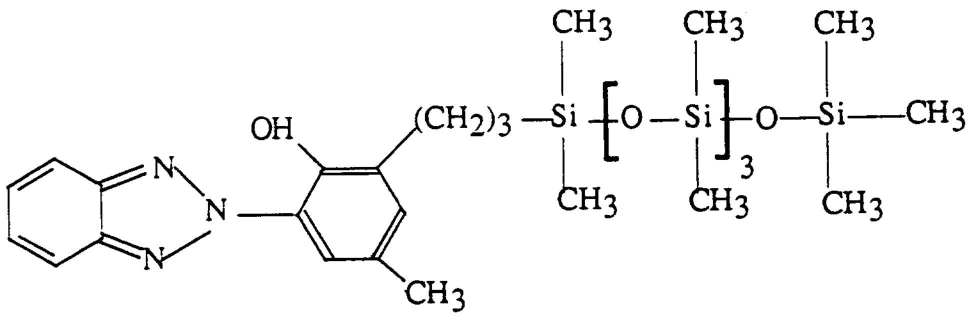

| | | | |
|---|---|---|---|
| selon la demande EP 0388218. | | | |
| - Décaméthylcyclopentasiloxane | | 25,8 | g |
| - Mélange de diméthiconol (13%), d'octaméthylcyclotétrasiloxane et de décaméthylcyclopentasiloxane (87%), vendu sous la dénomination Q2-1401 par la Société DOW CORNING | | 54,2 | g |
| - Benzoate d'alcools en $C_{12}$-$C_{15}$ vendu sous la dénomination FINSOLV TN par la Société WITCO | | 7,9 | g |
| - Huile de tournesol | | 0,5 | g |
| - Beurre de karité | | 0,1 | g |
| - Acétate de vitamine E | | 0,5 | g |
| - Conservateurs qs | | | |
| - Parfum qs | | | |
| - Alcool éthylique absolu | qsp | 100 | g |

EXEMPLE 3

On prépare une huile solaire transparente de composition suivante :

| | | | |
|---|---|---|---|
| - Polydiméthylsiloxane à greffon dérivé de benzophénone de formule : | | 6 | g |

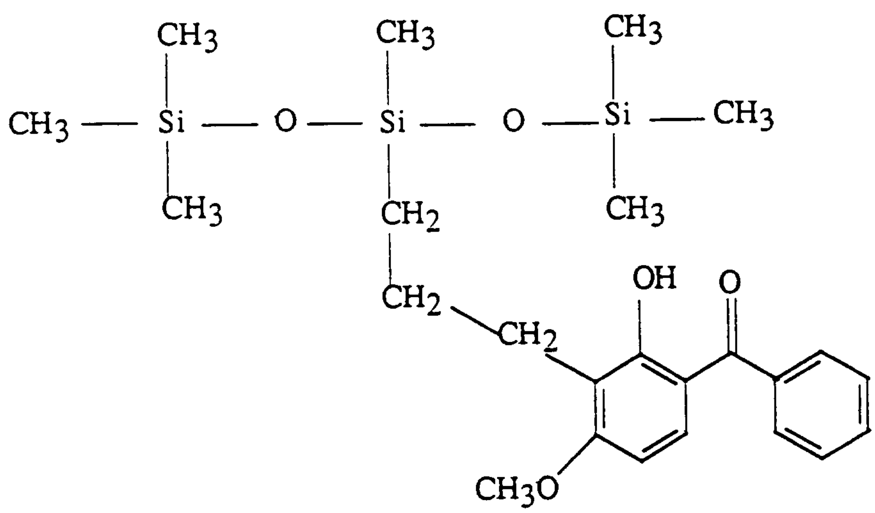

| | | | |
|---|---|---|---|
| - Décaméthylcyclopentasiloxane | | 25,8 | g |
| - Mélange de diméthiconol (13%), d'octaméthylcyclotétrasiloxane et de décaméthylcyclopentasiloxane (87%), vendu sous la dénomination Q2-1401 par la Société DOW CORNING | | 54,2 | g |
| - Benzoate d'alcools en $C_{12}$-$C_{15}$ vendu sous la dénomination FINSOLV TN par la Société WITCO | | 7,9 | g |
| - Huile de tournesol | | 0,5 | g |
| - Beurre de karité | | 0,1 | g |
| - Acétate de vitamine E | | 0,5 | g |
| - Conservateurs qs | | | |
| - Parfum qs | | | |
| - Alcool éthylique absolu | qsp | 100 | g |

Le polydiméthylsiloxane à greffon dérivé de benzophénone peut être remplacé par celui de formule :

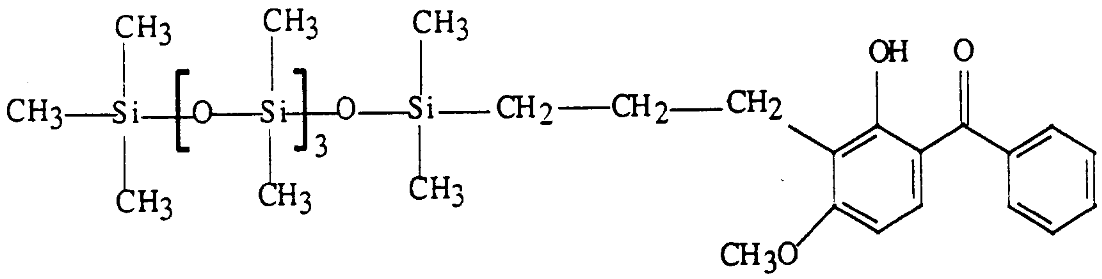

selon la demande de brevet français FR 2 657 351.

EXEMPLE 4

On prépare une huile solaire transparente de composition suivante :

| | | |
|---|---|---|
| - Polydiméthylsiloxane à greffon dérivé de 3-allyl-4,5-diméthoxybenzalmalonate de di-(2-éthylhexyle) de formule : | | 2 g |

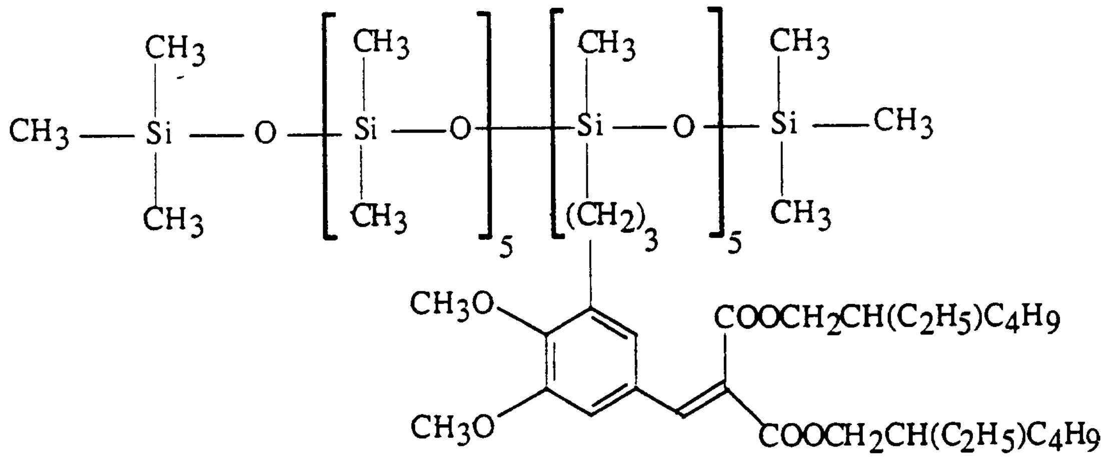

| | | |
|---|---|---|
| selon la demande de brevet EP 0392882 | | |
| - Décaméthylcyclopentasiloxane | | 29,8 g |
| - Mélange de diméthiconol (13%), d'octaméthylcyclotétrasiloxane et de décaméthylcyclopentasiloxane (87%), vendu sous la dénomination Q2-1401 par la Société DOW CORNING | | 54,2 g |
| - Benzoate d'alcools en $C_{12}$-$C_{15}$ vendu sous la dénomination FINSOLV TN par la Société WITCO | | 7,9 g |
| - Huile de tournesol | | 0,5 g |
| - Beurre de karité | | 0,1 g |
| - Acétate de vitamine E | | 0,5 g |
| - Conservateurs qs | | |
| - Parfum qs | | |
| - Alcool éthylique absolu | qsp | 100 g |

Cette huile peut être utilisée pour la protection de la peau ou des cheveux vis-à-vis du rayonnement UV.

EXEMPLE 5

On prépare une huile solaire transparente de composition suivante :

| | | | |
|---|---|---|---|
| - Polydiméthylsiloxane à greffon dérivé de benzotriazole de formule : | | 2 | g |

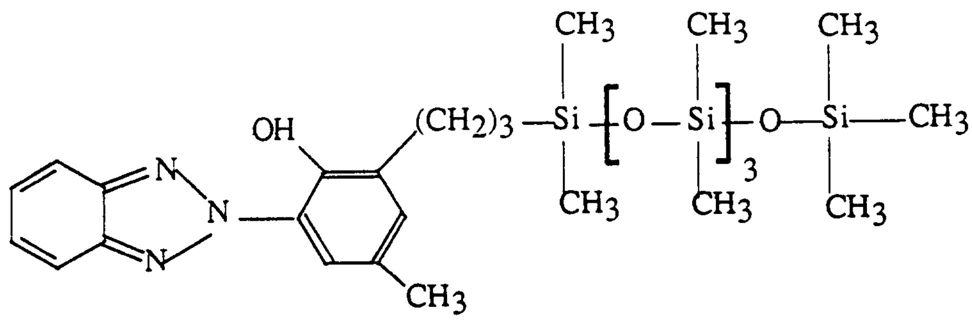

| | | | |
|---|---|---|---|
| selon la demande de brevet EP 0388218. | | | |
| - Décaméthylcyclopentasiloxane | | 24 | g |
| - Mélange de gomme polydiméthyldiphényl-siloxane à groupements terminaux diméthyl-vinylsilyloxy (PM 600.000) (15%), d'octa-méthylcyclotétrasiloxane (42,5%) et de décaméthylcyclopentasiloxane (42,5%) | | 60 | g |
| - Benzoate d'alcools en $C_{12}$-$C_{15}$ vendu sous la dénomination FINSOLV TN par la Société WITCO | | 7,9 | g |
| - Huile de tournesol | | 0,5 | g |
| - Beurre de karité | | 0,1 | g |
| - Acétate de vitamine E | | 0,5 | g |
| - Conservateurs qs | | | |
| - Parfum qs | | | |
| - Alcool éthylique absolu | qsp | 100 | g |

Cette huile peut être utilisée pour la protection de la peau ou des cheveux vis-à-vis du rayonnement UV.

EXEMPLE 6

On prépare une huile solaire transparente de composition suivante :

| | | | |
|---|---|---|---|
| - Polydiméthylsiloxane à greffon dérivé de benzotriazole de formule : | | 10 | g |

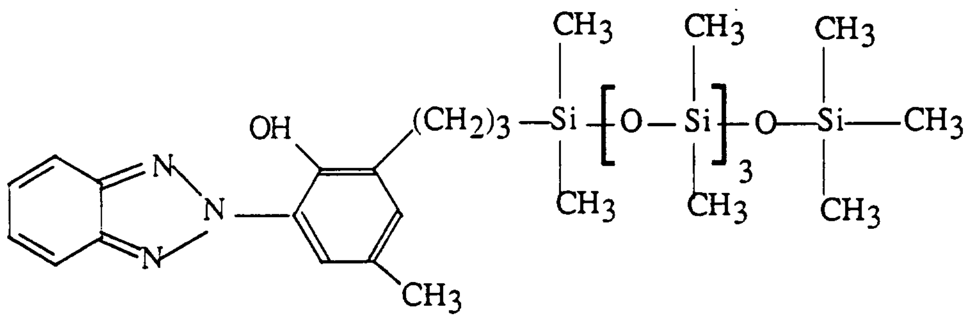

| | | | |
|---|---|---|---|
| selon la demande de brevet EP 0388218. | | | |
| - Décaméthylcyclopentasiloxane | | 21,8 | g |
| - Mélange de gomme polydiméthylsiloxane (15%) et de décaméthylcyclopentasiloxane (85%), vendu sous la dénomination SF 1214 SILICON FLUID par la Société GENERAL ELECTRIC | | 54,2 | g |
| - Benzoate d'alcools en $C_{12}$-$C_{15}$ vendu sous la dénomination FINSOLV TN par la Société WITCO | | 7,9 | g |
| - Huile de tournesol | | 0,5 | g |
| - Beurre de karité | | 0,1 | g |
| - Acétate de vitamine E | | 0,5 | g |
| - Conservateurs qs | | | |
| - Parfum qs | | | |
| - Alcool éthylique absolu | qsp | 100 | g |

EXEMPLE 7

On prépare une huile solaire transparente de composition suivante :

- Polydiméthylsiloxane à greffon dérivé de 3-allyl 4,5-diméthoxybenzalmalonate de (2-éthylhexyle) de formule : 2 g

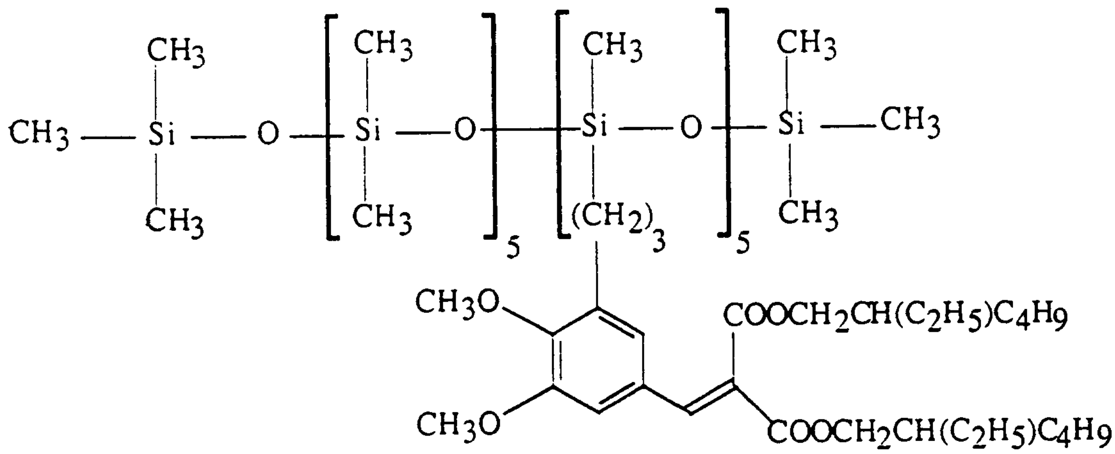

selon la demande de brevet EP 0392882

| | | | |
|---|---|---|---|
| - Décaméthylcyclopentasiloxane | | 25 | g |
| - Mélange de diméthiconol (13%), d'octaméthylcyclotétrasiloxane et de décaméthylcyclopentasiloxane (87%), vendu sous la dénomination Q2-1401 par la Société DOW CORNING | | 60 | g |
| - Ester d'acides gras ramifiés et d'alcools gras saturés en $C_{16}$-$C_{18}$, vendu sous la dénomination CETIOL SN DEO par la Société HENKEL | | 4 | g |
| - Monococoate de 2-éthyl hexyle | | 4 | g |
| - Conservateurs | qs | | |
| - Parfum | qs | | |
| - Alcool éthylique absolu | qsp | 100 | g |

Cette huile peut être utilisée pour la protection de la peau ou des cheveux vis-à-vis du rayonnement UV.

EXEMPLE 8

On prépare une émulsion solaire sous forme de gel crème de composition suivante :

- Mélange de polydiméthylsiloxanes à greffons 4'-triméthylèneoxy 3-benzylidènecamphre et 4'-oxy 3-benzylidènecamphre, préparé selon la demande EP 0335 777, ayant pour formule : 2,6 g

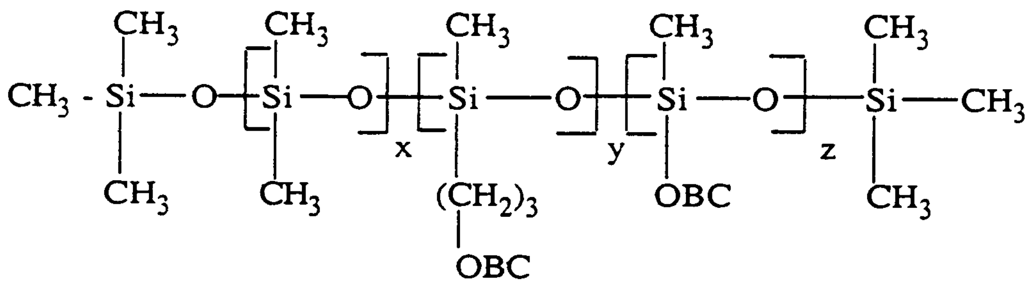

constitué d'environ :
- 55% de polydiméthylsiloxane dans lequel x = 0 à 20, y = 1 à 4, z = 0
- 5% de polydiméthylsiloxane dans lequel x = 0 à 21, y = 0, z = 1 à 4
- 40% de polydiméthylsiloxane dans lequel x = 0 à 16, y = 1 à 3, z = 1 à 3,

BC désignant le radical 3-benzylidènecamphre
- Décaméthylcyclopentasiloxane 4,98 g
- Mélange de diméthiconol (13%), d'octaméthylcyclotétrasiloxane et de décaméthylcyclopentasiloxane (87%), vendu sous la dénomination Q2-1401 par la Société DOW CORNING 10,84 g
- Benzoate d'alcools en $C_{12}$-$C_{15}$ vendu sous la dénomination FINSOLV TN par la Société WITCO 1,58 g
- Copolymère d'acide acrylique et d'acrylate d'alkyle ($C_{10}$-$C_{30}$) réticulé, vendu sous la dénomination PEMULEN TR1 par la Société GOODRICH (totalement neutralisé par la triéthanolamine) 0,3 g (avant neutralisation)

| | | | | |
|---|---|---|---|---|
| - Glycérine | | | 3 | g |
| - Conservateurs | qs | | | |
| - Parfum | qs | | | |
| - Eau | | qsp | 100 | g |

Cette émulsion peut être utilisée pour la protection de la peau ou des cheveux vis-à-vis du rayonnement UV.

EXEMPLE 9

On prépare une émulsion solaire de composition suivante :

- Mélange de polydiméthylsiloxanes à greffons 4'-triméthylèneoxy 3-benzylidènecamphre et 4'-oxy 3-benzylidènecamphre, préparé selon la demande EP 0335 777, ayant pour formule : 2,6 g

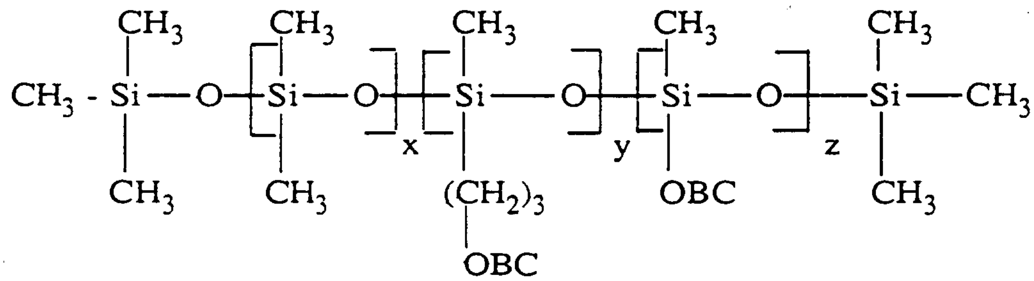

constitué d'environ :

- 55% de polydiméthylsiloxane dans lequel x = 0 à 20, y = 1 à 4, z = 0
- 5% de polydiméthylsiloxane dans lequel x = 0 à 21, y = 0, z = 1 à 4
- 40% de polydiméthylsiloxane dans lequel x = 0 à 16, y = 1 à 3, z = 1 à 3,

BC désignant le radical 3-benzylidènecamphre

- Décaméthylcyclopentasiloxane 4,98 g
- Mélange de diméthiconol (13%), d'octaméthylcyclotétrasiloxane et de décaméthylcyclopentasiloxane (87%), vendu sous la dénomination Q2-1401 par la Société DOW CORNING 10,84 g
- Benzoate d'alcools en $C_{12}$-$C_{15}$ vendu sous la dénomination FINSOLV TN par la Société WITCO 1,58 g
- Mélange de stéarate de glycérol et de stéarate de polyéthylèneglycol à 100 moles d'oxyde d'éthylène, vendu sous la dénomination ARLACEL 165 par la Société ICI 2 g
- Copolymère d'acide acrylique et d'acrylate d'alkyle ($C_{10}$-$C_{30}$) réticulé, vendu sous la dénomination PEMULEN TR1 par la

| | | | |
|---|---|---|---|
| Société GOODRICH (totalement neutralisé par la triéthanolamine) | | | 0,3 g (avant neutralisation) |
| - Conservateurs | qs | | |
| - Parfum | qs | | |
| - Eau | | qsp | 100 g |

Cette émulsion peut être utilisée pour la protection de la peau ou des cheveux vis-à-vis du rayonnement UV.

EXEMPLE 10

On prépare une huile solaire transparente de composition suivante :

- Mélange de polydiméthylsiloxanes à greffons 4'-triméthylèneoxy 3-benzylidènecamphre et 4'-oxy 3-benzylidènecamphre, préparé selon la demande EP 0335 777, ayant pour formule : 3 g

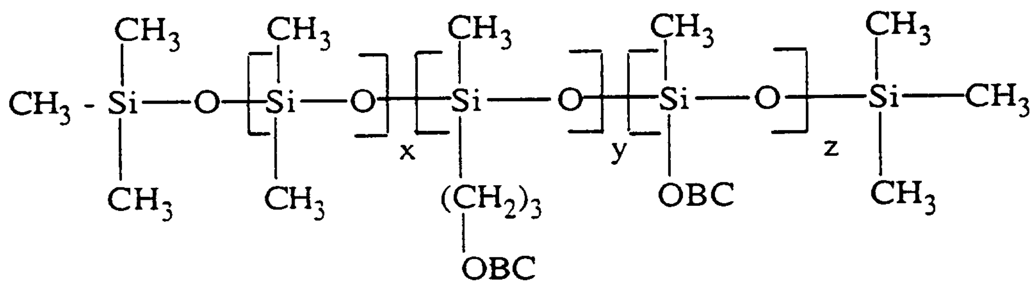

constitué d'environ :
- 55% de polydiméthylsiloxane dans lequel x = 0 à 20, y = 1 à 4, z = 0
- 5% de polydiméthylsiloxane dans lequel x = 0 à 21, y = 0, z = 1 à 4
- 40% de polydiméthylsiloxane dans lequel x = 0 à 16, y = 1 à 3, z = 1 à 3,

BC désignant le radical 3-benzylidènecamphre
- Polydiméthylsiloxane à greffon dérivé de benzotriazole de formule : 3 g

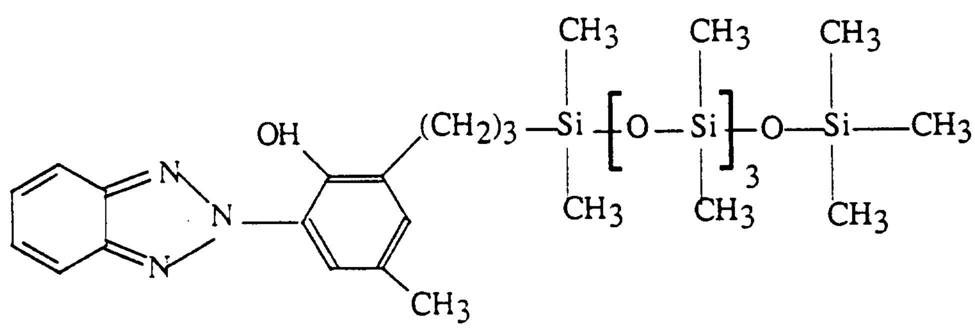

- Décaméthylcyclopentasiloxane 20 g
- Mélange de diméthiconol (13%), d'octaméthylcyclotétrasiloxane et de décaméthylcyclopentasiloxane (87%), vendu sous la dénomination Q2-1401 par la Société DOW CORNING 59,4 g

| | | | | |
|---|---|---|---|---|
| - Benzoate d'alcools en $C_{12}$-$C_{15}$, vendu sous la dénomination FINSOLV TN par la Société WITCO | | | 7,9 | g |
| - Huile de tournesol | | | 0,5 | g |
| - Beurre de karité | | | 0,1 | g |
| - Acétate de vitamine E | | | 0,5 | g |
| - Conservateurs | qs | | | |
| - Parfum | qs | | | |
| - Alcool éthylique absolu | | qsp | 100 | g |

**Revendications**

**1.** Huile cosmétique transparente et photostable filtrant le rayonnement ultraviolet, caractérisée par le fait qu'elle comprend au moins un organopolysiloxane filtrant les rayons ultraviolets choisi parmi ceux à groupements dérivés du benzylidènecamphre, de benzalmalonate, de benzophénone et de benzotriazole et un mélange de deux composants (A) et (B) constitué de :
(A) une silicone volatile
(B) une huile de silicone de viscosité cinématique supérieure à $10^{-1}$ $m^2/s$ à 25 °C ou une gomme de silicone.

**2.** Huile cosmétique filtrante selon la revendication 1, caractérisée par le fait que l'organopolysiloxane filtrant les rayons UV encore appelé "silicone filtre", comporte dans sa molécule au moins une unité de formule :

$$X-\overset{\displaystyle R_a}{\overset{|}{Si}}-O_{\frac{3-a}{2}} \qquad (I)$$

dans laquelle :
R désigne un groupe hydrocarboné saturé ou insaturé en $C_1$-$C_{30}$, un groupe hydrocarboné halogéné en $C_1$-$C_8$ ou un groupe triméthylsilyloxy;
a = 1 ou 2 ;
X = - A - Y
où A représente un radical divalent hydrocarboné aliphatique ou aromatique comportant au moins 2 atomes de carbone et renfermant éventuellement un ou plusieurs atomes d'oxygène;
Y représente le reste d'une molécule filtrant le rayonnement ultra- violet du type benzylidènecamphre, benzalmalonate, benzotriazole ou benzophénone,
au moins 40% en nombre des radicaux R étant des radicaux méthyle.

**3.** Huile cosmétique filtrante selon la revendication 2, caractérisée par le fait que le diorganopolysiloxane comporte en outre des unités ayant pour formules :

$$R_b-Si-O_{\frac{4-b}{2}} \quad (II) \quad ; \quad Z-\overset{\displaystyle R_a}{\overset{|}{Si}}-O_{\frac{3-a}{2}} \quad (III)$$

dans lesquelles :

a et R ont la même signification que dans la revendication 2;
b est un nombre entier égal à 1, 2 ou 3;
Z = OY, Y ayant la même signification que dans la revendication 2,
au moins 40% en nombre des radicaux R étant des radicaux méthyle.

**4.** Huile cosmétique filtrante selon l'une quelconque des revendications 1 à 3, caractérisée par le fait que le nombre total d'unités de formules (I), (II) et (III) est inférieur ou égal à 250 et de préférence compris entre 2 et 50.

**5.** Huile cosmétique filtrante selon l'une quelconque des revendications 1 à 4, caractérisée par le fait que le reste Y d'une molécule filtrant le rayonnement ultraviolet présent dans l'unité de formule (I) ou (III) du diorganopolysiloxane, est choisi parmi les restes de benzylidènecamphre non substitué ou substitué sur le noyau benzénique par des radicaux hydroxy, alkyle en $C_1$-$C_6$ ou alcoxy en $C_1$-$C_6$; de benzalmalonate de dialkyle en $C_1$-$C_8$, non substitué ou éventuellement substitué sur le noyau benzénique par des radicaux hydroxy, alkyle en $C_1$-$C_6$ ou alcoxy en $C_1$-$C_6$; de 2-(2'-hydroxyphényl)-benzotriazole non substitué ou portant sur l'un des noyaux aromatiques des substituants alkyle en $C_1$-$C_8$, halogène, alcoxy, carboxy, hydroxy ou amino; et de 2-hydroxybenzophénone non substituée ou portant d'autres substituants : alkyle en $C_1$-$C_8$, alcoxy en $C_1$-$C_8$, alcényloxy en $C_2$-$C_8$ ou hydroxy.

**6.** Huile cosmétique filtrante selon l'une quelconque des revendications 1 à 5, caractérisée par le fait qu'elle contient 0,5 à 13% en poids, et de préférence 1 à 8% en poids de silicone filtre à groupements dérivés du benzylidènecamphre, de benzalmalonate, de benzophénone ou de benzotriazole.

**7.** Huile cosmétique filtrante selon l'une quelconque des revendications 1 à 6, caractérisée par le fait qu'elle contient 67 à 99,5% en poids, et de préférence 75 à 80% en poids, du mélange (A + B) de silicone volatile (A) et d'huile de silicone de viscosité supérieure à $10^{-1}$ $m^2/s$ ou de gomme de silicone (B).

**8.** Huile cosmétique filtrante selon l'une quelconque des revendications 1 à 7, caractérisée par le fait que le mélange (A + B) est défini par un rapport pondéral B/A compris entre 0,07 et 0,2.

**9.** Huile cosmétique filtrante selon l'une quelconque des revendications 1 à 8, caractérisée par le fait que la silicone volatile (A) est choisie parmi les silicones cycliques comportant de 3 à 7 atomes de silicium, et de préférence parmi l'octaméthylcyclotétrasiloxane et le décaméthylcyclopentasiloxane ou leur mélange, et les silicones volatiles linéaires comportant 2 à 9 atomes de silicium et possédant une viscosité inférieure ou égale à $5.10^{-6}$ $m^2/s$ à 25°C.

**10.** Huile cosmétique filtrante selon l'une quelconque des revendications 1 à 9, caractérisée par le fait que les huiles de silicones de viscosité cinématique supérieure à $10^{-1}$ $m^2/s$ et les gommes de silicone (B) sont choisies parmi les polydiorganosiloxanes à groupements terminaux triméthylsilyle ou à groupements terminaux diméthylvinylsilyloxy dans le cas des gommes poly [(diméthylsiloxane)/-(diphénylsiloxane)].

**11.** Huile cosmétique filtrante selon l'une quelconque des revendications 1 à 10, caractérisée par le fait que les huiles de silicone ont une viscosité cinématique comprise entre $2.10^{-1}$ $m^2/s$ et $25.10^{-1}$ $m^2/s$ à 25°C.

**12.** Huile cosmétique filtrante selon l'une quelconque des revendications 1 à 10, caractérisée par le fait que les gommes de silicone ont une masse moléculaire comprise entre 200.000 et 1.000.000 et sont utilisées en mélange avec des silicones volatiles selon la revendication 9.

**13.** Huile cosmétique filtrante selon l'une quelconque des revendications 1 à 12, caractérisée par le fait qu'elle contient, outre la silicone filtre et le mélange (A + B), une proportion pondérale inférieure à 20% d'un mélange de solvants organiques constitué par un mélange d'alcool éthylique et d'un ester choisi parmi un benzoate d'alcools en $C_{12}$-$C_{15}$, le palmitate d'isopropyle, le myristate d'isopropyle, le monococoate de 2-éthylhexyle, le dipélargonate de propylèneglycol, un ester d'acides gras ramifiés et d'alcools gras saturés en $C_{16}$-$C_{18}$, un ester d'acides gras et de glycérol, un ester d'acides gras

(caprylique-caprique) et de propylèneglycol, ou des mélanges de ces composés.

**14.** Huile cosmétique filtrante selon la revendication 13, caractérisée par le fait qu'elle contient une silicone filtre à groupements dérivés du benzylidènecamphre, de benzalmalonate, de benzophénone et de benzotriazole, associée à un mélange d'une silicone volatile (A) et d'une gomme de silicone (B), ainsi qu'un mélange d'alcool éthylique et d'un benzoate d'alcools en $C_{12}$-$C_{15}$.

**15.** Utilisation de l'huile cosmétique filtrante selon l'une quelconque des revendications 1 à 14, à titre de phase grasse pour la préparation d'une émulsion cosmétique filtrante du type huile-dans-eau ou eau-dans-huile.

**16.** Emulsion cosmétique filtrante du type huile-dans-eau ou eau-dans-huile, caractérisée par le fait qu'elle comprend, à titre de phase grasse, l'huile cosmétique filtrante selon l'une quelconque des revendications 1 à 14.

**17.** Procédé cosmétique de protection de l'épiderme humain et des cheveux contre le rayonnement ultraviolet, caractérisé par le fait qu'il consiste à appliquer sur la peau ou les cheveux une quantité efficace de l'huile cosmétique filtrante selon l'une quelconque des revendications 1 à 14.

## Claims

**1.** Transparent and photostable cosmetic oil screening out ultraviolet radiation, characterized in that it comprises at least one organopolysiloxane screening out ultraviolet rays, chosen from those containing groups derived from benzylidenecamphor, from benzalmalonate, from benzophenone and from benzotriazole, and a mixture of two components (A) and (B) consisting of:
(A) a volatile silicone
(B) a silicone oil of kinematic viscosity greater than $10^{-1}$ $m^2/s$ at 25 °C or a silicone gum.

**2.** Cosmetic screening oil according to Claim 1, characterized in that the organopolysiloxane screening out UV rays, also referred to as "silicone screening agent", contains in its molecule at least one unit of formula:

$$X - \overset{R_a}{\overset{|}{Si}} - O_{\frac{3-a}{2}} \qquad (I)$$

in which:

R denotes a saturated or unsaturated $C_1$-$C_{30}$ hydrocarbon group, a halogenated $C_1$-$C_8$ hydrocarbon group or a trimethylsilyloxy group;

a = 1 or 2;

X = - A - Y where A represents a bivalent aliphatic or aromatic hydrocarbon radical containing at least 2 carbon atoms and optionally including one or more oxygen atoms;

Y represents the residue of a molecule screening out ultraviolet radiation, of the benzylidenecamphor, benzalmalonate, benzotriazole or benzophenone type,

at least 40% of the radicals R, in numerical terms, being methyl radicals.

**3.** Cosmetic screening oil according to Claim 2, characterized in that the diorganopolysiloxane contains, in addition, units of the formulae:

$$R_b - Si - O_{\frac{4-b}{2}} \quad (II) \quad ; \quad Z - \overset{\overset{\displaystyle R_a}{|}}{Si} - O_{\frac{3-a}{2}} \quad (III)$$

in which:

a and R have the same meaning as in Claim 2;
b is an integer equal to 1, 2 or 3;
Z = OY, Y having the same meaning as in Claim 2,
at least 40% of the radicals R, in numerical terms, being methyl radicals.

**4.** Cosmetic screening oil according to any one of Claims 1 to 3, characterized in that the total number of units of formulae (I), (II) and (III) is less than or equal to 250, and preferably between 2 and 50.

**5.** Cosmetic screening oil according to any one of Claims 1 to 4, characterized in that the residue Y of a molecule screening out ultraviolet radiation, present in the unit of formula (I) or (III) of the diorganopolysiloxane, is chosen from a benzylidenecamphor residue, unsubstituted or substituted on the benzene ring with hydroxyl, $C_1$-$C_6$ alkyl or $C_1$-$C_6$ alkoxy radicals; a $C_1$-$C_8$ dialkyl benzalmalonate residue, unsubstituted or optionally substituted on the benzene ring with hydroxyl, $C_1$-$C_6$ alkyl or $C_1$-$C_6$ alkoxy radicals; a 2-(2'-hydroxyphenyl)-benzotriazole residue, unsubstituted or bearing $C_1$-$C_8$ alkyl, halogen, alkoxy, carboxyl, hydroxyl or amino substituents on one of the aromatic rings; and a 2-hydroxybenzophenone residue, unsubstituted or bearing other substituents: $C_1$-$C_8$ alkyl, $C_1$-$C_8$ alkoxy, $C_2$-$C_8$ alkenyloxy or hydroxyl.

**6.** Cosmetic screening oil according to any one of Claims 1 to 5, characterized in that it contains 0.5 to 13% by weight, and preferably 1 to 8% by weight, of silicone screening agent containing groups derived from benzylidenecamphor, from benzalmalonate, from benzophenone or from benzotriazole.

**7.** Cosmetic screening oil according to any one of Claims 1 to 6, characterized in that it contains 67 to 99.5% by weight, and preferably 75 to 80% by weight, of the mixture (A + B) of volatile silicone (A) and silicone oil of viscosity greater than $10^{-1}$ $m^2/s$ or silicone gum (B).

**8.** Cosmetic screening oil according to any one of Claims 1 to 7, characterized in that the mixture (A + B) is defined by a B/A weight ratio of between 0.07 and 0.2.

**9.** Cosmetic screening oil according to any one of Claims 1 to 8, characterized in that the volatile silicone (A) is chosen from cyclic silicones containing from 3 to 7 silicon atoms, and preferably from octamethylcyclotetrasiloxane and decamethylcyclopentasiloxane or a mixture thereof, and linear volatile silicones containing 2 to 9 silicon atoms and possessing a viscosity not exceeding $5 \times 10^{-6}$ $m^2/s$ at 25°C.

**10.** Cosmetic screening oil according to any one of Claims 1 to 9, characterized in that the silicone oils of kinematic viscosity greater than $10^{-1}$ $m^2/s$ and the silicone gums (B) are chosen from polydiorganosiloxanes containing terminal trimethylsilyl groups or containing terminal dimethylvinylsilyloxy groups in the case of poly[(dimethylsiloxane)/(diphenylsiloxane)] gums.

**11.** Cosmetic screening oil according to any one of Claims 1 to 10, characterized in that the silicone oils have a kinematic viscosity of between $2 \times 10^{-1}$ $m^2/s$ and $25 \times 10^{-1}$ $m^2/s$ at 25°C.

**12.** Cosmetic screening oil according to any one of Claims 1 to 10, characterized in that the silicone gums have a molecular mass of between 200,000 and 1,000,000, and are used mixed with volatile silicones according to Claim 9.

**13.** Cosmetic screening oil according to any one of Claims 1 to 12, characterized in that it contains, in addition to the silicone screening agent and the mixture (A + B), a weight proportion of less than 20%

of a mixture of organic solvents consisting of a mixture of ethyl alcohol and an ester chosen from a benzoate of $C_{12}$-$C_{15}$ alcohols, isopropyl palmitate, isopropyl myristate, 2-ethylhexyl monococoate, propylene glycol dipelargonate, an ester of branched fatty acids and saturated $C_{16}$-$C_{18}$ fatty alcohols, an ester of fatty acids and glycerol, an ester of fatty acids (caprylic/capric) and propylene glycol, or mixtures of these compounds.

**14.** Cosmetic screening oil according to Claim 13, characterized in that it contains a silicone screening agent containing groups derived from benzylidenecamphor, from benzalmalonate, from benzophenone and from benzotriazole, in combination with a mixture of a volatile silicone (A) and a silicone gum (B), as well as a mixture of ethyl alcohol and a benzoate of $C_{12}$-$C_{15}$ alcohols.

**15.** Use of the cosmetic screening oil according to any one of Claims 1 to 14, as fatty phase for the preparation of a cosmetic screening emulsion of the oil-in-water or water-in-oil type.

**16.** Cosmetic screening emulsion of the oil-in-water or water-in-oil type, characterized in that it comprises, as fatty phase, the cosmetic screening oil according to any one of Claims 1 to 14.

**17.** Cosmetic process for protecting the human epidermis or hair against ultraviolet radiation, characterized in that it consists in applying to the skin or hair an effective amount of the cosmetic screening oil according to any one of Claims 1 to 14.

**Patentansprüche**

**1.** Durchsichtiges und photostabiles kosmetisches Öl, das die ultraviolette Strahlung filtert,
dadurch **gekennzeichnet,** daß
es mindestens ein die ultravioletten Strahlen filterndes Organopolysiloxan, ausgewählt aus denjenigen mit Gruppierungen, die von Benzylidenkampfer, Benzalmalonat, Benzophenon und Benzotriazol abgeleitet sind, sowie eine Mischung aus den beiden Bestandteilen (A) und (B) aus:
(A) einem flüchtigen Silikon und
(B) einem Silikonöl einer kinematischen Viskosität von mehr als $10^{-1}$ $m^2/s$ bei 25 °C oder einem Silikon-Gummi enthält.

**2.** Filterndes kosmetisches Öl gemäß Anspruch 1,
dadurch **gekennzeichnet,** daß
das die UV-Strahlen filternde Organopolysiloxan, das auch "Silikon-Filter" genannt wird, in seinem Molekül mindestens eine Einheit der Formel aufweist:

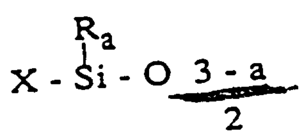

(I)

worin gilt:
R bedeutet eine gesättigte oder ungesättigte $C_{1-30}$-Kohlenwasserstoffgruppe, eine halogenierte $C_{1-8}$-Kohlenwasserstoffgruppe oder eine Trimethylsilyloxygruppe;
a = 1 oder 2;
X = -A-Y,
worin A einen aliphatischen oder aromatischen Zweiwertigen Kohlenwasserstoffrest darstellt, der mindestens zwei Kohlenstoffatome aufweist und gegebenenfalls ein oder mehrere Sauerstoffatome enthält;
Y stellt den Rest eines die ultraviolette Strahlung filternden Moleküls des Benzylidenkampfer-, Benzalmalonat-, Benzotriazol- oder Benzophenon-Typs dar,
wobei mindestens 40% der Anzahl der Reste R Methylreste sind.

**3.** Filterndes kosmetisches Öl gemäß Anspruch 2,
dadurch **gekennzeichnet,** daß
das Diorganopolysiloxan ausserdem Einheiten mit den Formeln aufweist:

$$R_b-Si-O_{\frac{4-b}{2}} \quad (II) \quad ; \quad Z-\overset{R_a}{\overset{|}{Si}}-O_{\frac{3-a}{2}} \quad (III)$$

in denen gilt:
a und R haben dieselbe Bedeutung wie in Anspruch 2,
b ist eine ganze Zahl von 1, 2 oder 3,
Z = OY, worin Y dieselbe Bedeutung wie in Anspruch 2 hat,
wobei mindestens 40% der Anzahl der Reste R Methylreste sind.

**4.** Kosmetisches filterndes Öl gemäß einem der Ansprüche 1 bis 3,
dadurch **gekennzeichnet,** daß
die Gesamtzahl an Einheiten der Formeln (I), (II) und (III) weniger als oder gleich 250 und vorzugsweise 2 bis 50 beträgt.

**5.** Kosmetisches filterndes Öl gemäß jedem der Ansprüche 1 bis 4,
dadurch **gekennzeichnet,** daß
der Rest Y eines die ultraviolette Strahlung filternden Molekül, der in der Einheit der Formel (I) oder (III) des Diorganopolysiloxans vorliegt, aus Resten von Benzylidenkampfer, der am Benzolkern mit Hydroxy-, $C_{1-6}$-Alkyl- oder $C_{1-6}$-Alkoxyresten substituiert ist oder nicht, aus Resten von $C_{1-8}$-Dialkylbenzalmalonat, das am Benzolkern mit Hydroxy-, $C_{1-6}$-Alkyl- oder $C_{1-6}$-Alkoxyresten gegebenenfalls substituiert ist, aus Resten von 2-(2'-Hydroxyphenyl)benzotriazol, das nicht substituiert ist oder an einem der aromatischen Kerne $C_{1-8}$-Alkyl-, Halogen-, Alkoxy-, Carboxy-, Hydroxy- oder Amino-Substituenten aufweist, sowie aus Resten von 2-Hydroxybenzophenon ausgewählt ist, das nicht substituiert ist oder weitere Substituenten aufweist: $C_{1-8}$-Alkyl-, $C_{1-8}$-Alkoxy-, $C_{2-8}$-Alkenyloxy- oder Hydroxy-Substituenten.

**6.** Kosmetisches filterndes Öl gemäß jedem der Ansprüche 1 bis 5,
dadurch **gekennzeichnet,** daß
es 0,5 bis 13, vorzugsweise 1 bis 8, Gew.% Silikon-Filter mit von Benzylidenkampfer, Benzalmalonat, Benzophenon oder Benzotriazol abgeleiteten Gruppierungen enthält.

**7.** Kosmetisches filterndes Öl gemäß jedem der Ansprüche 1 bis 6,
dadurch **gekennzeichnet,** daß
es 67 bis 99,5, vorzugsweise 75 bis 80, Gew.% der Mischung (A + B) aus flüchtigem Silikon (A) und Silikonöl einer Viskosität von mehr als $10^{-1}$ $m^2/s$ oder Silikon-Gummi (B) enthält.

**8.** Kosmetisches filterndes Öl gemäß jedem der Ansprüche 1 bis 7,
dadurch **gekennzeichnet,** daß
die Mischung (A + B) durch ein Gewichtsverhältnis B/A von 0,07 bis 0,2 definiert ist.

**9.** Kosmetisches filterndes Öl gemäß jedem der Ansprüche 1 bis 8,
dadurch **gekennzeichnet,** daß
das flüchtige Silikon (A) aus zyklischen Silikonen mit 3 bis 7 Siliziumatomen und vorzugsweise aus Octamethylcyclotetrasiloxan und Decamethylcyclopentasiloxan oder aus deren Mischungen und aus flüchtigen linearen Silikonen ausgewählt ist, die 2 bis 9 Siliziumatome aufweisen und eine Viskosität von weniger als oder gleich 5 x $10^{-6}$ $m^2/s$ bei 25 °C besitzen.

**10.** Kosmetisches filterndes Öl gemäß jedem der Ansprüche 1 bis 9,
dadurch **gekennzeichnet,** daß
die Silikonöle einer kinematischen Viskosität von mehr als $10^{-1}$ $m^2/s$ und die Silikon-Gummmiprodukte (B) aus Polydiorganosiloxanen mit endständigen Trimethylsilylgruppierungen oder mit endständigen Dimethylvinylsilyloxygruppierungen im Fall der Poly((dimethylsiloxan)/(diphenylsiloxan))-Gummiprodukte ausgewählt sind.

**11.** Kosmetisches filterndes Öl gemäß jedem der Ansprüche 1 bis 10,
dadurch **gekennzeichnet,** daß
die Silikonöle eine kinematische Viskosität von 2 bis 25 x $10^{-1}$ $m^2/s$ bei 25 °C aufweisen.

**12.** Kosmetisches filterndes Öl gemäß jedem der Ansprüche 1 bis 10,
dadurch **gekennzeichnet,** daß
die Silikon-Gummiprodukte eine Molekularmasse von 200 000 bis 1 000 000 aufweisen und in Mischung mit flüchtigen Silikonen gemäß Anspruch 9 verwendet werden.

**13.** Kosmetisches filterndes Öl gemäß jedem der Ansprüche 1 bis 12,
dadurch **gekennzeichnet,** daß
es, ausser dem Silikon-Filter und der Mischung (A + B), einen Gewichtsanteil von weniger als 20% einer Mischung organischer Lösungsmittel aus einer Mischung aus Ethylalkohol und einem Ester enthält, der aus einem $C_{12-15}$-Alkylbenzoat, Isopropylpalmitat, Isopropylmyristat, 2-Ethylhexylmonococoat, Propylenglycoldipelargonat, einem Ester von verzweigten Fettsäuren und gesättigten $C_{16-18}$-Fettalkoholen, einem Ester von Fettsäuren und Glycerin, einem Ester von Fettsäuren (Capron-Caprinsäuren) und Propylenglycol oder aus Mischungen dieser Verbindungen ausgewählt ist.

**14.** Kosmetisches filterndes Öl gemäß Anspruch 13,
dadurch **gekennzeichnet,** daß
es einen Silikon-Filter mit von Benzylidenkampfer, Benzalmalonat, Benzophenon und Benzotriazol abgeleiteten Gruppierungen, zusammen mit einer Mischung aus einem flüchtigen Silikon (A) und einem Silikon-Gummi (B), sowie eine Mischung aus Ethylalkohol und einem $C_{12-15}$-Alkylbenzoat enthält.

**15.** Verwendung des kosmetischen filternden Öls gemäß jedem der Ansprüche 1 bis 14 als Fettphase zur Herstellung einer kosmetischen filternden Emulsion des Öl-in-Wasser- oder Wasser-in-Öl-Typs.

**16.** Kosmetische filternde Emulsion des Öl-in-Wasser- oder Wasser-in-Öl-Typs,
dadurch **gekennzeichnet,** daß
sie, als Fettphase, das kosmetische filternde Öl gemäß jedem der Ansprüche 1 bis 14 enthält.

**17.** Kosmetisches Verfahren zum Schutz der menschlichen Epidermis und der Haare vor ultravioletter Strahlung,
dadurch **gekennzeichnet,** daß
man auf die Haut oder die Haare eine wirksame Menge eines kosmetischen filternden Öls gemäß jedem der Ansprüche 1 bis 14 aufbringt.